# EUROPÄISCHE PATENTSCHRIFT

(11) **EP 0 395 849 B1**
(45) Veröffentlichungstag und Bekanntmachung des Hinweises auf die Patenterteilung: **19.04.1995**
(21) Anmeldenummer: 90103661.6
(22) Anmeldetag: 26.02.1990
(51) Int. Cl.: C07F 9/10, A61K 31/10, A61K 31/685

(54) **/3-(C16-C18)alkansulfinyl- und sulfonyl-2-methoxymethyl-propyl/-(2-trimethylammonio-ethyl)phosphate, Verfahren zu deren Herstellung und diese Verbindungen enthaltende Arzneimittel**
[3-(C16-C18)alkane sulfinyl- and sulfonyl-2-methoxymethyl-propyl]-(2-trimethylammonio-ethyl) phosphates, process for their preparation and medicaments containing these
Phosphates de [3-(C16-C18)alcane sulfinyl- et sulfonyl-2-méthoxyméthyl-propyl]-(2-triméthylammonio-éthyl), leur procédé de préparation et médicaments les contenant

(30) Priorität: 04.03.1989 DE 3906952
(43) Veröffentlichungstag der Anmeldung: 07.11.1990
(73) Patentinhaber: BOEHRINGER MANNHEIM GMBH, 68298 Mannheim (DE)
(72) Erfinder: Bosies, Elmar, Dr., D-6940 Weinheim (DE); Herrmann, Dieter, Dr., D-6900 Heidelberg (DE); Palke, Wulf, Dr., D-6140 Bensheim (DE)

(56) Entgegenhaltungen:
- DE-A- 2 619 686
- DE-A- 2 619 715
- DE-C- 2 009 341
- US-A- 4 444 766

## Beschreibung

Die vorliegende Erfindung betrifft [3-(C₁₆-C₁₈)alkansulfinyl- und sulfonyl-2-methoxymethyl-propyl]-(2-trimethylammonio-ethyl)phosphate, deren pharmakologisch unbedenklichen Salze, Verfahren zu deren Herstellung sowie Arzneimittel, die diese Verbindungen enthalten.

Propyl-(trialkylammonio-alkyl)phosphate, die in 3-Stellung durch Alkylmercapto-, Alkylsulfinyl- und Alkylsulfonylreste substituiert sind, sind Gegenstand der EP 050 327. Beschrieben sind insbesondere Verbindungen, die in 3-Stellung eine Alkylmercaptogruppe tragen, so z.B. das (3-Hexadecylmercapto-2-methoxymethyl-propyl)-(2-trimethylammonio-ethyl)phosphat (Ilmofosine), das hervorragende antitumorale Eigenschaften besitzt.

Es wurde nun gefunden, daß die analogen Sulfinyl- und Sulfonyl-Verbindungen sowie die entsprechenden Heptadecyl- und Octadecylderivate, die in der EP 050 327 nicht beschrieben sind, aber unter den breiten Anspruch fallen, überraschenderweise in-vivo eine noch bessere Wirkung als das Ilmofosine aufweisen, insbesondere die beiden Verbindungen (3-Hexadecansulfonyl-2-methoxymethyl-propyl)-(2-trimethylammonio-ethyl)phosphat und (2-Methoxymethyl-3-octadecansulfonyl-propyl)-(2-trimethylammonio-ethyl)phosphat.

Gegenstand der vorliegenden Erfindung sind demnach Verbindungen der allgemeinen Formel I 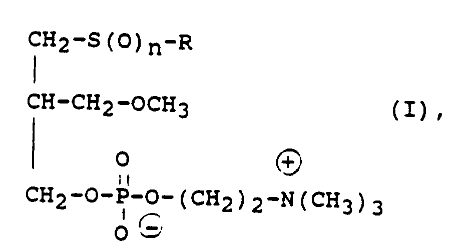
in der
n = 1 oder 2 und
R eine geradkettige, gesättigte Alkylkette mit 16 - 18 Kohlenstoffatomen bedeuten, deren Stereoisomere sowie deren pharmakologisch unbedenklichen Salze.

Bevorzugt sind hierbei Verbindungen der allgemeinen Formel I, bei denen n = 1 oder 2 und R den Hexadecyl- bzw. Octadecylrest bedeuten.

Besonders bevorzugt sind das (3-Hexadecansulfonyl-2-methoxymethyl-propyl)-(2-trimethylammonio-ethyl)phosphat und das (2-Methoxymethyl-3-octadecansulfonyl-propyl)-(2-trimethylammonio-ethyl)phosphat.

Gegenstand der Erfindung sind ferner sämtliche stereoisomeren Verbindungen der Formel I, die z.B. aufgrund des asymmetrischen Kohlenstoffatoms oder der Sulfoxidgruppe anfallen.

Die Verbindungen der allgemeinen Formel I werden nach an sich bekannten Methoden hergestellt, vorzugsweise in dem man
I) eine Verbindung der allgemeinen Formel II, die als Racemat oder als Enantiomeres vorliegen kann, 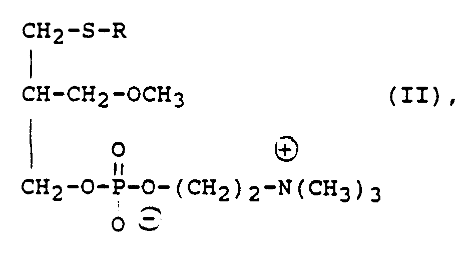 in der R die oben angegebene Bedeutung hat, mit einem Oxidationsmittel behandelt, oder
II) eine Verbindung der allgemeinen Formel III, die als Racemat oder als Enantiomeres vorliegen kann, 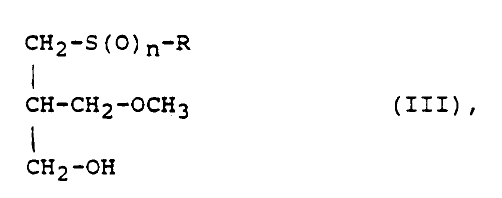 in der n und R die oben angegebenen Bedeutungen haben,
   a) mit einer Verbindung der allgemeinen Formel IV 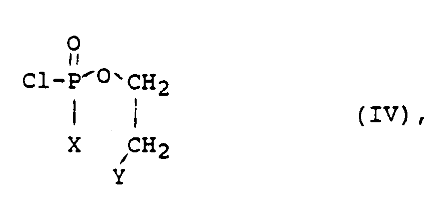 in der X = Chlor, Y = Brom oder X und Y zusammen Sauerstoff bedeuten, in Gegenwart eines säurebindenden Mittels umsetzt und das Reaktionsprodukt mit Trimethylamin behandelt, wobei im Falle von X = Chlor eine selektive Hydrolyse der Aminierung vorausgeht, oder
   b) in eine Verbindung der allgemeinen Formel V überführt 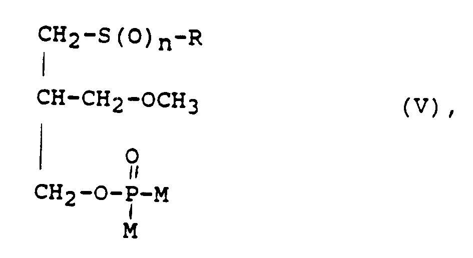 in der n und R die oben angegebenen Bedeutungen haben und M = Hydroxy, Chlor, Brom oder Alkylmercapto bedeutet, und diese mit einer Verbindung der allgemeinen Formel VI

      HO-CH₂CH₂-T (VI),

      in der T = Chlor, Brom oder die Gruppe N(CH₃)₃⁺ Hal⁻ darstellt, wobei Hal⁻ Chlorid, Bromid oder Iodid sein soll, umsetzt und für den Fall, daß T = Chlor oder Brom ist, das so erhaltene Zwischenprodukt anschließend mit Trimethylamin quaternisiert, oder
   c) mit einer Verbindung der allgemeinen Formel VII 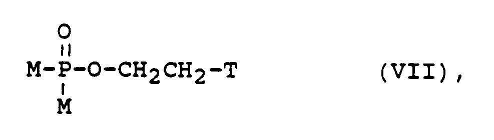 in der M und T die oben angegebenen Bedeutungen haben, in Gegenwart eines säure- oder wasserbindenden Mittels umsetzt und gegebenenfalls in das innere Salz überführt.

Bei Verfahren I wird als Oxidationsmittel vorzugsweise 30proz. Wasserstoffperoxid in Eisessig verwendet. Bei Einsatz von äquimolaren Mengen erhält man die Sulfoxide, mit einem drei- bis fünffachen Überschuß an Oxidationsmittel gelangt man zu den entsprechenden Sulfonen. Man kann zur Oxidation aber auch sehr gut organische Peroxide wie z.B. m-Chlorperbenzoesäure einsetzen, wobei man inerte Lösungsmittel wie z.B. Methylenchlorid verwendet. Die Reaktionstemperatur liegt zwischen 0 und 50°C, vorzugsweise bei 20°C.

Die als Ausgangsverbindung eingesetzten Thioether der allgemeinen Formel II sind in der EP 050 327 beschrieben oder lassen sich nach den dort angegebenen Methoden herstellen.

Die bei Verfahren II angegebenen Methoden sind ebenfalls in der EP 050 327 beschrieben. Die hierbei eingesetzten Sulfoxide bzw. Sulfone der allgemeinen Formel III lassen sich analog der im Verfahren I beschriebenen Oxidation aus den entsprechenden Thioethern, die ebenfalls in der EP 050 327 beschrieben sind oder sich nach den dort angegebenen Verfahren herstellen lassen, gewinnen.

Die für die Synthese der enantiomerenreinen Verbindungen benötigten optisch aktiven Zwischenstufen der allgemeinen Formel III lassen sich z.B. nach folgender Methode herstellen. Ein als Racemat vorliegender Alkohol der allgemeinen Formel III wird mit Hilfe eines optisch aktiven Hilfsreagenz in ein Diastereomerenpaar überführt, dies mit Hilfe säulenchromatographischer Methoden getrennt und hieraus jeweils durch Abspaltung des Hilfsreagenzes der entantiomerenreine Alkohol der allgemeinen Formel III gewonnen. So kann man als Hilfsreagenz optisch aktive Säuren wie z.B. die(R)- oder (S)-2-Phenylpropionsäure oder die (S)-Camphansäure mit dem Alkohol der allgemeinen Formel III verestern. Nach der Trennung der Diastereomeren werden die Ester sauer, verzugsweise jedoch alkalisch verseift. Dasselbe Verfahren kann auch zur Trennung der Thioetheralkohole, die als Vorstufen für die Synthese der Verbindungen der allgemeinen Formel III eingesetzt werden, verwendet werden. Diese so getrennten Thioetheralkohole können dann analog der in Verfahren II beschriebenen Methode zu optisch aktiven Verbindungen der allgemeinen Formel II, die als Ausgangsmaterial für Verfahren I genommen werden können, umgesetzt werden.

Die pharmakologisch verträglichen Salze erhält man in üblicher Weise, z.B. durch Neutralisation der Verbindungen der Formel I mit nichttoxischen anorganischen oder organischen Säuren wie z.B. Salzsäure, Schwefelsäure, Phosphorsäure, Bromwasserstoffsäure, Essigsäure, Milchsäure, Zitronensäure, Äpfelsaure, Salicylsäure, Malonsäure, Maleinsäure oder Bernsteinsäure.

Die erfindungsgemäßen neuen Substanzen der allgemeinen Formel I und ihre Salze können in flüssiger oder fester Form enteral oder parenteral appliziert werden. Hierbei kommen alle üblichen Applikationsformen in Frage, beispielsweise Tabletten, Kapseln, Dragees, Sirupe, Lösungen, Suspensionen etc. Als Injektionsmedium kommt vorzugsweise Wasser zur Anwendung, welches die bei Injektionslösungen üblichen Zusätze wie Stabilisierungsmittel, Lösungsvermittler und Puffer enthält.

Derartige Zusätze sind z.B. Tartrat- und Citratpuffer, Ethanol, Komplexbildner (wie Ethylendiamintetraessigsäure und deren nichttoxische Salze) und hochmolekulare Polymere (wie flüssiges Polyethylenoxid) zur Viskositätsregulierung. Flüssige Trägerstoffe für Injektionslösungen müssen steril sein und werden vorzugsweise in Ampullen abgefüllt. Feste Trägerstoffe sind z.B. Stärke, Lactose, Mannit, Methylcellulose, Talkum, hochdisperse Kieselsäuren, hochmolekulare Fettsäuren (wie Staerinsäure), Gelatine, Agar-Agar, Calciumphosphat, Magnesiumstearat, tierische und pflanzliche Fette und feste hochmolekulare Polymere (wie Polyethylenglykole). Für orale Applikation geeignete Zubereitungen können gewünschtenfalls Geschmacks- und Süßstoffe enthalten.

Die Dosierung kann von verschiedenen Faktoren, wie Applikationsweise, Spezies, Alter und/oder individuellen Zuständen abhängen. Die täglich zu verabreichenden Dosen liegen bei etwa 0.05-100 mg/kg Körpergewicht.

Die Wirkung der Substanzen wurde in einem in-vivo-Modell an dem Methylcholanthren-induzierten Fibrosarkom der Maus geprüft.

### Versuchsbeschreibung

Weiblichen CB₆F₁-Mäusen wurden am Tag 0 1x 10⁵ Meth-A-Zellen i.c. gespritzt. Die Therapie erfolgte täglich ab Tag 1 bis zum Tag 21 mit 2.5, 10 und 40 mg/kg p.o. Anzahl der Tiere pro Gruppe: 10. Als Parameter wurden bestimmt:
1) Das Tumorvolumen am Tag 7, 14, 21 und 28 nach Tumorzellinokulation.
2) Das Tumorgewicht am Tag 28.

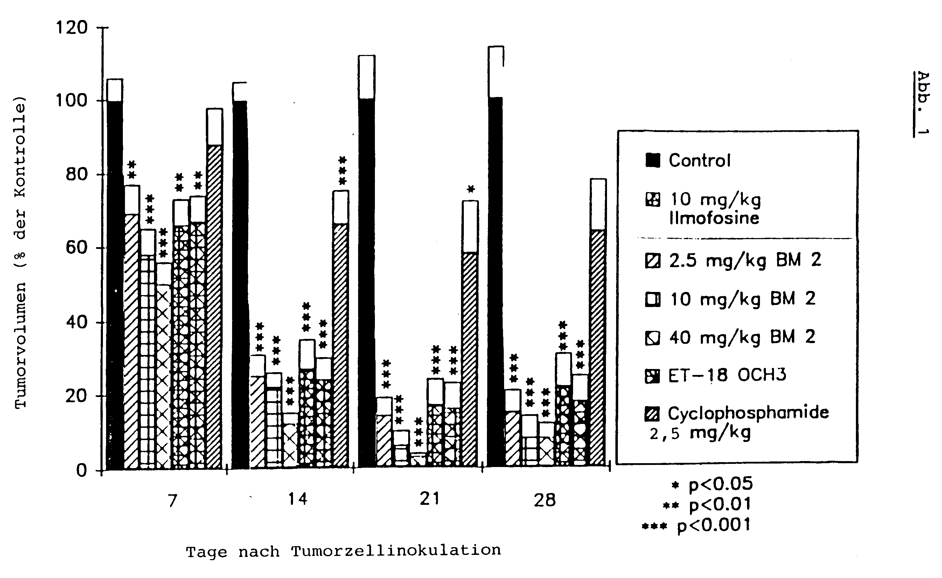

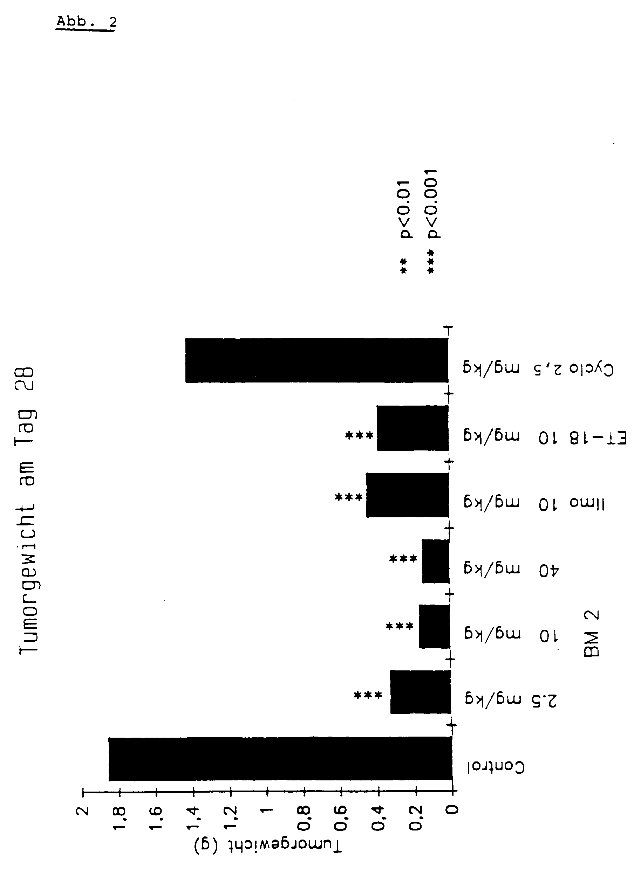

Als Vergleichssubstanz wurden die am Schwefel nicht oxidierte Verbindung (3-Hexadecylmercapto-2-methoxymethyl-propyl)-(2-trimethylammonio-ethyl)phosphat (Ilmofosine) aus der EP 050 327 und die Verbindung (3-Octadecyloxy-2-methoxy-propyl)-(2-trimethylammonio-ethyl)phosphat (ET-18-OCH₃) gewählt, als Positivkontrolle Cyclophosphamid mitgeführt **(s. Abbildung 1 und 2)**.

Bei der Betrachtung des Tumorvolumens sieht man deutlich, daß 2.5 mg BM 2 [= (3-Hexadecansulfonyl-2-methoxymethyl-propyl)-(2-trimethylammonio-ethyl)phosphat] dieselbe Wirksamkeit zeigt wie 10 mg Ilmofosine. Das Tumorgewicht am Tag 28 zeigt dasselbe Ergebnis. BM 2 ist deutlich wirksamer als Ilmofosine.

### Beispiel 1

### (3-Hexadecansulfonyl-2-methoxymethyl)-propyl)-(2-trimethylammonio-ethyl)phosphat

Zu 7.56 g (14 mmol) (3-Hexadecylmercapto-2-methoxymethyl-propyl)-(2-trimethylammonio-ethyl)phosphat in 70 ml Eisessig gibt man 4.2 ml (42 mmol) 30proz. Wasserstoffperoxid und läßt bei Raumtemperatur rühren. Nach 24 h gibt man noch einmal 1.4 ml Wasserstoffperoxid zu und läßt insgesamt 72 h rühren. Die Lösung wird am Rotavapor bei 20°C eingeengt, der Rückstand mit Diethylether versetzt und über Nacht im Kühlschrank stehengelassen. Man dekantiert die Etherlösung von einem zähen Niederschlag ab, löst diesen in 1 l Wasser und läßt die Lösung gefriertrocknen. Man erhält so 5.2 g = 65 % der gewünschten Verbindung in amorpher Form mit 1 mol Wasser; R_{f}-Wert auf einer RP-18-Kieselgelplatte mit dem Fließmittelgemisch Aceton(7)/Wasser(3)/0.1 % Eisessig: 0.35; SO-Derivat: 0.39; Ausgangsmaterial: 0.28

### In analoger Weise wurden durch Verwendung des (-)- bzw. (+)-enantiomeren Thioethers die folgenden Sulfonylderivate hergestellt:

a) (-)-(3-Hexadecansulfonyl-2-methoxymethyl-propyl)-(2-trimethylammonio-ethyl)phosphat
b) (+)-(3-Hexadecansulfonyl-2-methoxymethyl-propyl)-(2-trimethylammonio-ethyl)phosphat
c) (3-Octadecansulfonyl-2-methoxymethyl-propyl)-(2-trimethylammonio-ethyl)phosphat
d) (-)-(3-Octadecansulfonyl-2-methoxymethyl-propyl)-(2-trimethylammonio-ethyl)phosphat
e) (+)-(3-Octadecansulfonyl-2-methoxymethyl-propyl)-(2-trimethylammonio-ethyl)phosphat

### Beispiel 2

### (3-Hexadecansulfinyl-2-methoxymethyl-propyl)-(2-trimethylammonio-ethyl)phosphat

Zu 10.8 g (20 mmol) (3-Hexadecylmercapto-2-methoxymethyl-propyl)-(2-trimethylammonio-ethyl)phosphat in 100 ml Eisessig gibt man 2 ml (20 mmol) 30proz. Wasserstoffperoxid und läßt die Lösung 144 h bei Raumtemperatur stehen. Die Lösung wird am Rotavapor eingeengt, der Rückstand jeweils zweimal in Toluol aufgenommen und wieder eingeengt. Anschließend verrührt man den Rückstand mit 250 ml Aceton, filtriert von wenig Ungelöstem ab und läßt das Filtrat langsam verdunsten. Nachdem ca. die Hälfte des Acetons verdunstet ist, fällt ein Niederschlag aus. Man saugt ab und erhält 3.0 g = 27 % des gewünschten Produktes, Fp: 235-240°C; R_{f}-Wert auf einer RP-18-Kieselgelplatte mit dem Fließmittelgemisch Aceton(7)/Wasser(3)/ 0.1% Eisessig: 0.39; SO₂-Derivat: 0.35; Ausgangsmaterial: 0.28

### In analoger Weise werden durch Verwendung des (-)-bzw. - (+)-enantiomeren Thioethers die folgenden Sulfoxide hergestellt:

a) (-)-(3-Hexadecansulfinyl-2-methoxymethyl-propyl)-(2-trimethylammonio-ethyl)phosphat
b) (+)-(3-Hexadecansulfinyl-2-methoxymethyl-propyl)-(2-trimethylammonio-ethyl)phosphat

### Beispiel 3

Analog Beispiel 2 wird durch Einsatz des entsprechenden Octadecylderivates hergestellt das

### (3-Octadecansulfinyl-2-methoxymethyl-propyl)-(2-trimethylammonio-ethyl)phosphat

und durch Verwendung der entsprechenden (-)- bzw. (+)-enantiomeren Thioether das
a) (-)-(3-Octadecansulfinyl-2-methoxymethyl-propyl)-(2-trimethylammonio-ethyl)phosphat
b) (+)-(3-Octadecansulfinyl-2-methoxymethyl-propyl)-(2-trimethylammonio-ethyl)phosphat

## Patentansprüche

1. Verbindungen der Formel I 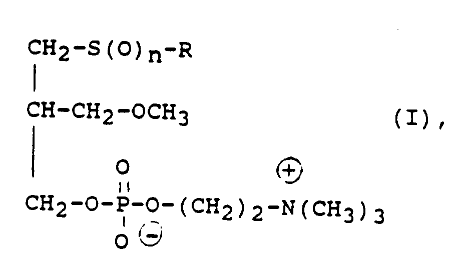 in der
n die Zahlen 1 oder 2, und
R eine geradkettige, gesättigte Alkylkette mit 16-18 Kohlenstoffatomen
bedeutet,
deren Stereoisomere sowie deren pharmakologisch unbedenkliche Salze.

2. Verbindungen (3-Hexadecylsulfonyl-2-methoxymethylpropyl)-(2-trimethylammonio-ethyl)phosphat und 3-Octadecylsulfonyl-2-methoxymethylpropyl)-(2-trimethylammonio-ethyl)phosphat,
sowie deren Stereoisomere nach Anspruch 1.

3. Verfahren zur Herstellung von Verbindungen der Formel I 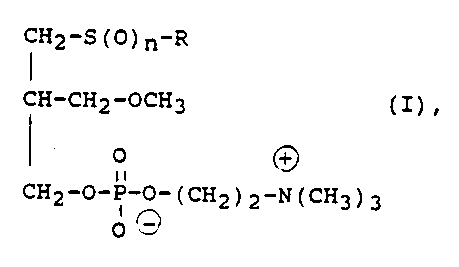 in der
n die Zahlen 1 oder 2, und
R eine geradkettige, gesättigte Alkylkette mit 16-18 Kohlenstoffatomen
bedeutet,
deren Stereoisomere sowie deren pharmakologisch unbedenkliche Salze dadurch gekennzeichnet, daß man in an sich bekannter Weise
I) eine Verbindung der allgemeinen Formel II, die als Racemat oder als Enantiomeres vorliegen kann, 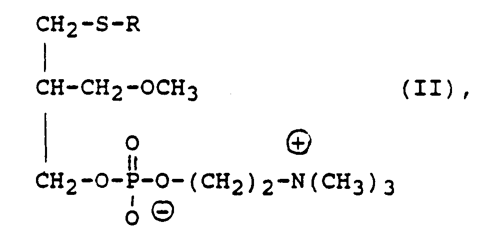 in der R die oben angegebene Bedeutung hat, mit einem Oxidationsmittel behandelt, oder
II) eine Verbindung der allgemeinen Formel III, die als Racemat oder als Enantiomeres vorliegen kann, 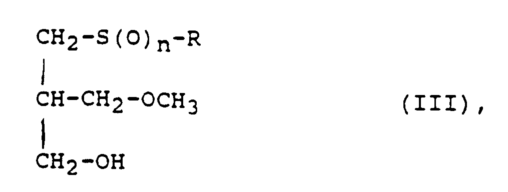 in der n und R die oben angegebenen Bedeutungen haben,
a) mit einer Verbindung der allgemeinen Formel IV 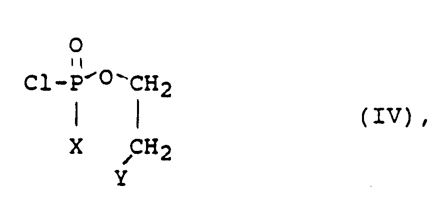 in der X = Chlor, Y = Brom oder X und Y zusammen Sauerstoff bedeuten, in Gegenwart eines säurebindenden Mittels umsetzt und das Reaktionsprodukt mit Trimethylamin behandelt, wobei im Falle von X = Chlor eine selektive Hydrolyse der Aminierung vorausgeht,
oder
b) in eine Verbindung der allgemeinen Formel V überführt 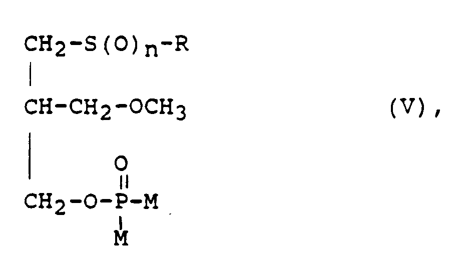 in der n und R die oben angegebenen Bedeutungen haben und M = Hydroxy, Chlor, Brom oder Alkylmercapto bedeutet, und diese mit einer Verbindung der allgemeinen Formel VI
HO-CH₂CH₂-T (VI),
in der T = Chlor, Brom oder die Gruppe N(CH₃)₃⁺ Hal⁻ darstellt, wobei Hal⁻ Chlorid, Bromid oder Iodid sein soll, umsetzt und für den Fall, daß T = Chlor oder Brom ist, das so erhaltene Zwischenprodukt anschließend mit Trimethylamin quaternisiert,
oder
c) mit einer Verbindung der allgemeinen Formel VII 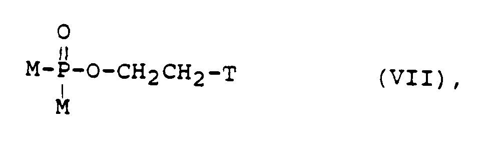 in der M und T die oben angegebenen Bedeutungen haben, in Gegenwart eines säure- oder wasserbindenden Mittels umsetzt und gegebenenfalls in das innere Salz überführt,
und anschließend gegebenenfalls die erhaltenen racemischen Verbindungen in optische Isomere und/oder pharmakologisch unbedenkliche Salze überführt.

4. Arzneimittel, enthaltend eine Verbindung gemäß Anspruch 1 oder 2 neben üblichen Träger- und Hilfsstoffen.

## Claims

1. Compounds of the formula I 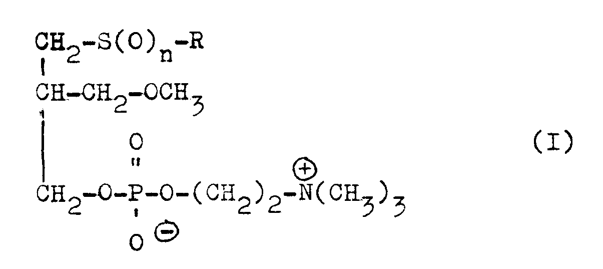 in which n signifies the numbers 1 or 2 and R a straight-chained, saturated alkyl chain with 16 - 18 carbon atoms, their stereoisomers, as well as their pharmacologically acceptable salts.

2. The compounds (3-hexadecylsulphonyl-2-methoxymethylpropyl)-(2-trimethylammonioethyl) phosphate and 3-octadecylsulphonyl-2-methoxymethylpropyl)-(2-trimethylammonioethyl) phosphate, as well as their stereoisomers according to claim 1.

3. Process for the preparation of compounds of the formula I 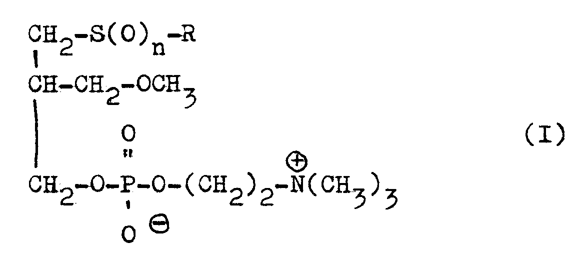 in which n signifies the numbers 1 or 2 and R a straight-chained, saturated alkyl chain with 16 - 18 carbon atoms, of their stereoisomers, as well as of their pharmacologically acceptable salts, characterised in that, in per se known manner, one
I) treats a compound of the general formula II, which can be present as racemate or as enantiomer 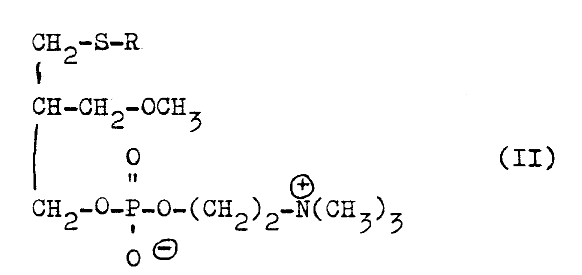 in which R has the above-given meaning, with an oxidation agent, or
II) reacts a compound of the general formula III, which can be present as racemate or as enantiomer 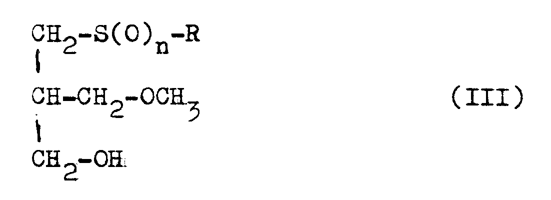 in which n and R have the above-given meanings,
a) with a compound of the general formula IV 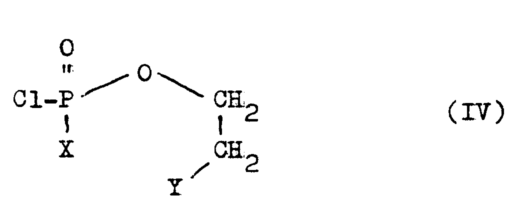 in which X = chlorine, Y = bromine or X and Y together signify oxygen, in the presence of an acid-binding agent and treats the reaction product with trimethylamine, whereby, in the case of X = chlorine, a selective hydrolysis precedes the amination, or
b) converts into a compound of the general formula V 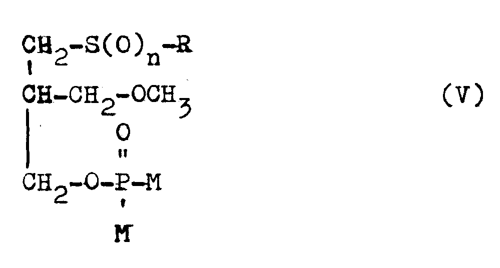 in which n and R have the above-given meanings and M = hydroxyl, chlorine, bromine or alkylmercapto, and reacts this with a compound of the general formula VI
HO-CH₂CH₂-T (VI)
in which T = chlorine, bromine or the group N(CH₃)₃^{⊕} Hal^{⊖}, whereby Hal⁻ is to be chloride, bromide or iodide, and, for the case that T = chlorine or bromine, subsequently quaternises the so obtained intermediate product with trimethylamine, or
c) reacts with a compound of the general formula VII 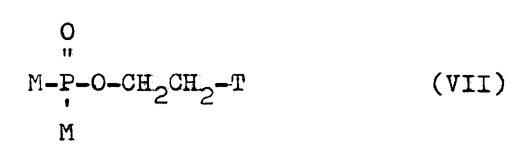 in which M and T have the above-given meanings, in the presence of an acid- or water-forming agent and possibly converts into the internal salt and subsequently possibly converts the racemic compounds obtained into optical isomers and/or pharmacologically acceptable salts.

4. Medicaments containing a compound according to claim 1 or 2, besides usual carrier and auxiliary materials.

## Revendications

1. Composés de formule I 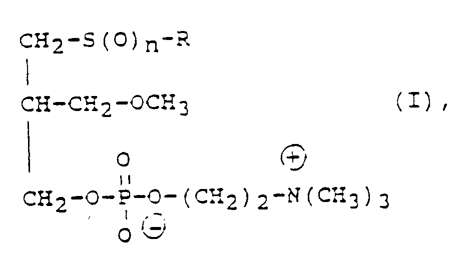 dans laquelle
n vaut 1 ou 2, et
R représente une chaîne alkylique linéaire, saturée, ayant 16-18 atomes de carbone,
leurs stéréoisomères ainsi que leurs sels pharmacologiquement acceptables.

2. Composés phosphate de (3-hexadécylsulfonyl-2-méthoxyméthylpropyl)-(2-triméthylammonioéthyle) et phosphate de (3-octadécylsulfonyl-2-méthoxyméthylpropyl)-(2-triméthylammonioéthyle),
ainsi que leurs stéréoisomères selon la revendication 1.

3. Procédé de préparation de composés de formule I 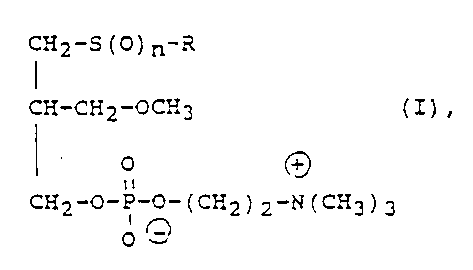 dans laquelle
n vaut 1 ou 2, et
R représente une chaîne alkylique linéaire, saturée, ayant 16-18 atomes de carbone,
leurs stéréoisomères ainsi que leurs sels pharmacologiquement acceptables, caractérisé en ce que, de manière connue en soi,
(I) un composé de formule générale II, qui peut être sous la forme de racémate ou d'énantiomère, 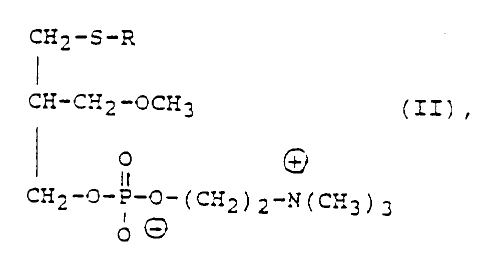 dans laquelle R a la signification mentionnée ci-dessus, est traité avec un agent oxydant, ou
(II) un composé de formule générale III, qui peut être sous la forme de racémate ou d'énantiomère, 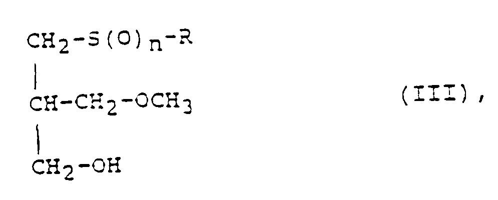 dans laquelle n et R ont les significations mentionnées ci-dessus,
a) est mis à réagir avec un composé de formule générale IV 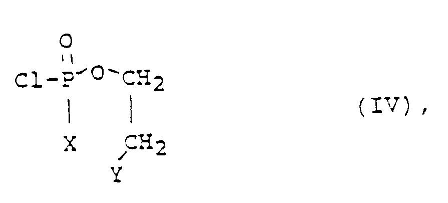 dans laquelle X = chlore, Y = brome, ou X et Y représentent ensemble un oxygène, en présence d'un agent fixant les acides, et le produit de la réaction est traité avec de la triméthylamine, une hydrolyse sélective de l'amination étant produite préalablement dans le cas où X = chlore,
ou
b) est transformé en un composé de formule générale V 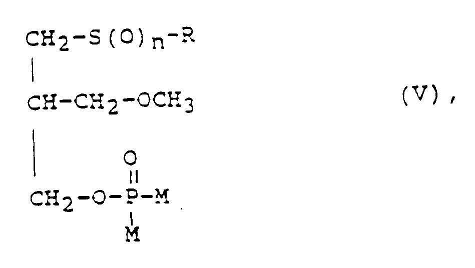 dans laquelle n et R ont les significations mentionnées ci-dessus, et M = hydroxy, chlore, brome ou alkylmercapto, et ce composé est mis à réagir avec un composé de formule générale VI
HO-CH₂-CH₂-T (VI),
dans laquelle T = le chlore, le brome ou le groupe N(CH₃)₃⁺Hal⁻, Hal⁻ représentant un chlorure, bromure ou iodure, et dans le cas où T = le chlore ou le brome, le produit intermédiaire ainsi obtenu est quaternisé ensuite avec la triméthylamine,
ou
c) est mis à réagir avec un composé de formule générale VII 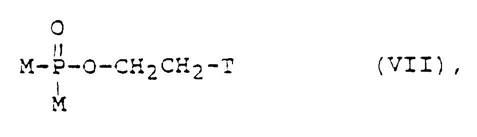 dans laquelle M et T ont les significations mentionnées ci-dessus, en présence d'un agent fixant les acides ou l'eau, et il est éventuellement transformé en son sel interne,
et ensuite, les mélanges racémiques obtenus sont transformés éventuellement en isomères optiques et/ou en sels pharmacologiquement acceptables.

4. Médicament, contenant un composé selon la revendication 1 ou 2, avec des véhicules et adjuvants usuels.
